Europäisches Patentamt

(19) European Patent Office

Office européen des brevets

(11) Numéro de publication: **0 004 516**
A2

(12) # DEMANDE DE BREVET EUROPEEN

(21) Numéro de dépôt: 79400194.1

(22) Date de dépôt: 27.03.79

(51) Int. Cl.²: **A 61 K 31/55**
// C07D281/02

(30) Priorité: 28.03.78 CH 3309/78

(43) Date de publication de la demande: 03.10.79
Bulletin 79/20

(84) Etats contractants désignés: **BE DE FR GB IT NL SE**

(71) Demandeur: **SCIENCE UNION ET Cie SOCIETE FRANCAISE DE RECHERCHE MEDICALE, 14 rue du Val d'Or, F-92150 Suresnes (FR)**

(72) Inventeur: **Malen, Charles, 3 Allée Traversière, F-94260 Fresnes (FR)**
Inventeur: **Poignant, Jean-Claude, Rue de la Fontaine St Mathieu, F-91440 Bures/Yvette (FR)**

(74) Mandataire: **Burtin, Jean-François, SCIENCE UNION ET Cie Service Brevets 22 rue Garnier, F-92200 Neuilly (FR)**

(54) Nouvelles compositions pharmaceutiques agissant sur la motricité gastro-intestinale.

(57) La présente invention a pour objet de nouvelles compositions pharmaceutiques agissant sur la motricité gastro-intestinale caractérisée en ce qu'elles renferment comme principe actif l'acide 7-[(3-chloro 6-méthyl 5,5-dioxo 6,11-dihydro dibenzo (c, f) thiazepin 1,2-11-yl) amino] heptanoïque sous forme racémique ou sous forme dédoublée ou un de ses sels, en association avec un véhicule ou un excipient inerte, non-toxique, pharmaceutiquement acceptable.

EP 0 004 516 A2

Nouvelles compositions pharmaceutiques agissant sur la
motricité gastro-intestinale

La présente invention a pour objet de nouvelles
compositions pharmaceutiques agissant sur la motricité gastro-
intestinale.

L'invention a plus particulièrement pour objet de
nouvelles compositions pharmaceutiques ayant pour principe
actif un dérivé de thiazépine en association ou en mélange
avec un excipient ou un véhicule inerte, non-toxique, pharmaceutiquement acceptable.

Spécifiquement l'invention a pour objet de nouvelles compositions pharmaceutiques renfermant comme principe
actif une thiazépine choisie dans le groupe constitué par
l'acide 7-[3-chloro 6-méthyl 5,5-dioxo 6,11-dihydro dibenzo
(c,f) thiazepin 1,2- 11-yl) amino] heptanoïque, un de ses
sels avec une base minérale ou organique thérapeutiquement
compatible et un de ses sels avec un acide minéral ou organique thérapeutiquement compatible, sous forme racémique ou
optiquement active.

Le choix du principe actif réside principalement
dans la voie d'administration et la forme pharmaceutique
utilisée. Il peut être avantageux d'utiliser un principe
actif insoluble dans l'eau mais facilement solubilisable
dans l'organisme comme l'acide 7-[3-chloro 6-methyl 5,5-
dioxo 6,11-dihydro dibenzo (c,f) thiazepin-1,2 11-yl) amino]
heptanoïque. Il peut aussi être avantageux d'utiliser un
dérivé soluble dans l'eau, facilement diffusible comme par
exemple un sel d'acide minéral ou organique, de préférence
un acide minéral fort ou bien un sel de métal alcalin de
préférence le sel de sodium ou de potassium ou un sel avec une amine mono- di- ou tri- alcoylée,comme par exemple la méthylamine, l'isopropylamine, la diéthylamine, l'amino-éthanol, le di (éthylamino) éthanol,le di (amino éthanol), la triéthylamine, la phényl méthylamine, la N-méthyl benzyl-amine, une hétéro cyclylalcoylamine comme par exemple la 2-(N-éthylamino) pyridine, une cycloalcoylamine comme la cyclopropylamine, une cycloalcoyl alcoylamine comme par exemple la 1-cyclopropyl 1-éthylamine, une bétaïne ou la choline.

Les sels hydrosolubles présentent un intérêt plus particulier pour la réalisation de formes injectables ou de formes ingérables.

Pour d'autres usages il peut être avantageux de disposer d'un principe actif peu soluble dans l'eau ou même pratiquement insoluble dans l'eau soit pour des raisons de sapidité soit pour diminuer la résorption du principe actif. Il conviendra donc d'utiliser un principe actif sous forme de sel avec une base minérale comme par exemple, avec une base alcalino-terreuse, avec l'alumine, avec la magnésie, avec le titane. avec une terre rare, un métal de la famille du fer, avec une base dérivant des métaux de transition ; ou bien avec une base organique susceptible de fournir des sels insolubles dans l'eau comme par exemple, des sels avec des bases de poids moléculaire plus élevé comme par exemple les arylamines, les arylalcoylamines, les hétérocyclyl alcoylamines, les alcényl-amines, les sels avec des protéines comme la clupéine, la salmine ou la protamine. On pourra également utiliser des sels avec les amino acides comme l'agmatine, l'arginine, la cana-valine, la créatine, la créatinine, la cystéine, la cystine, la proline, l'hydroxyproline. On pourra également utiliser des sels avec des dérivés d'amines biologiques comme la gramine, la O-méthylserine, la N-méthylvaline.

On pourra également former des sels insolubles dans l'eau avec des ammoniums quaternaires, notamment avec des aryl ou hétéro aryl trialcoylammonium comme par exemple le chlorure ou le bromure de benzalkonium, le bromure de cetrimonium, le chlorure de cetalkonium ou le bromure de cethexonium.

Parmi les sels d'addition avec un acide, on pourra citer comme sels hydrosolubles, les sels avec un acide minéral comme l'acide chlorhydrique, l'acide bromhydrique, l'acide sulfurique, l'acide nitrique, l'acide phosphorique ou l'acide iodique. Comme sels d'addition avec un acide, peu ou pas solubles dans l'eau ou les liquides aqueux, on pourra citer en particulier les sels avec les acides organiques comme par example l'acide acétique, l'acide valérique, l'acide caproïque, l'acide benzoïque, l'acide 3,5-dichlorobenzoïque, l'acide α-naphtoïque, l'acide pyrrolidone carboxylique, l'acide 2-méthylthiazole-5-carboxylique, l'acide 5-méthylthiazolidinyl-4 carboxylique, l'acide nicotinique, l'acide isonicotinique, l'acide benzène sulfonique, l'acide glucose-1-phosphorique ou l'acide embonique.

En vue de l'usage thérapeutique en médecine humaine ou animale, les compositions selon l'invention comprennent outre le principe actif, un ou plusieurs excipients inertes, un agent d'adhésion, ou des diluants, ou un des agents qui agissent pour accélérer ou retarder le délitement des formes solides, des agents d'aromatisation, des colorants, des émulsionnants ou des agents tensioactifs. On peut y ajouter également des agents de conservation, des stabilisants ou des agents anti-oxydants.

Le principe actif, l'acide 7-[3-chloro 6-methyl 5,5-dioxo 6,11-dihydro dibenzo (c,f) thiazepin 1,2-11-yl) amino] heptanoïque et ses sels est un produit connu, déjà décrit dans le brevet français 2 104 728 au nom de la

- 4 -

société demanderesse, portant sur la classe générale des acides dibenzothiazepinylamino alcanoïques. Ces composés sont décrits comme possédant des propriétés psychostimulantes, antidépressives, analgésiques, antitussives et antihistaminiques.

On a trouvé maintenant que l'acide 7-[(3-chloro 6-méthyl 5,5-dioxo 6,11-dihydro dibenzo (c,f) thiazepin 1,2-11-yl) amino] heptanoïque et ses sels manifestaient des propriétés pharmacologiques intéressantes et très spécifiques. On peut donc considérer ces composés comme une classe distincte de composés au sein de la vaste classe des acides (dibenzothiazepinylamino) alcanoïques antérieurement décrite.

Les études pharmacologiques et cliniques ont montré que l'acide 7-[(3-chloro 6-méthyl 5,5-dioxo 6,11-dihydro dibenzo (c,f) thiazepin 1,2-11-yl) amino] heptanoïque et ses sels présentaient une action antispasmodique importante, étaient capables de restaurer une motricité constante et bien coordonnée et ainsi de s'opposer à l'atonie de la musculature lisse tube digestif, présentait une action anti sérotonine très importante, directement mise en évidence sur la fibre lisse et protégeait l'animal contre l'ulcère produit par la contrainte. Cet effet protecteur ne s'accompagne pas d'effet anti-secrétoire et doit être attribué aux effets centraux des produits.

L'ensemble de ces propriétés peut être rattachée à des modifications des médiateurs chimiques cérébraux.

L'acide 7-[(3-chloro 6-méthyl 5,5-dioxo 6,11-dihydro dibenzo (c,f) thiazepin 1,2-11-yl) amino] heptanoïque produit une augmentation du taux d'acétylcholine cérébrale et produit une accélération du métabolisme de la dopamine et une baisse de la sérotonine.

En outre les compositions selon l'invention entraînent une tendance à la baisse du taux de prolactine. Ce phénomène s'oppose aux observations faites avec les antidépresseurs tricycliques qui augmentent nettement la prolactinémie. L'acide 7-[(3-chloro 6-méthyl 5,5-dioxo 6,11-dihydro dibenzo (c,f) thiazépine 1,2-11 yl) amino] heptanoïques est donc différent dans son mode d'action des dérivés tricycliques et des neurotropes. Il n'en possède pas les effets secondaires.

Cette constatation confirme l'existence d'une action originale du produit sur les structures activatrices de la musculature lisse viscérale.

Les compositions pharmaceutiques selon l'invention trouvent de ce fait un emploi pour le traitement des troubles de la motricité gastro-intestinale associés, ou non à un état dépressif, d'asthénie ou d'anxiété.

L'indication thérapeutique majeure des compositions pharmaceutiques à base d'acide 7-[(3-chloro 6-méthyl 5,5-dioxo 6,11-dihydro dibenzo (c,f) thiazepin 1,2-11-yl) amino] heptanoïque ou d'un de ses sels est le syndrome dyspeptique hyposténique. Les troubles du transit, les états nauséeux, les troubles digestifs des mégacolons congénitaux, les dyspepsies, l'atonie vésiculaire, la constipation et les troubles atypiques tels que ballonnement, pesanteur, somnolence post-prandiale.

0004516

- 6 -

Les compositions pharmaceutiques selon l'invention renferment entre 12 et 50mg de principe actif exprimé en acide 7-[(3-chloro 6-méthyl 5,5-dioxo 6,11-dihydro dibenzo (c,f) thiazepin 1,2-11-yl) amino] heptanoïque. Elles sont présentées sous l'une des formes convenant pour l'administration par voie parentérale, buccale, ou rectale.

On pourra citer comme formes pharmaceutiques actuellement préférées les comprimés nus ou enrobés, les dragées, les capsules, les gélules, les solutions, suspensions ou émulsions buvables, les gouttes ; les solutions ou suspensions injectables conditionnées en ampoules, flacons multidoses, les seringues auto-injectables, les suppositoires.

La posologie journalière chez l'adulte s'échelonne entre 12 et 75mg par jour depréférence, répartie en une, deux ou trois prises. Elle peut varier en fonction du poids et de l'âge du sujet, de l'indication thérapeutique et de la voie d'administration.

Les exemples suivants illustrent l'invention. Ils ne la limitent en aucune façon.

EXEMPLE I

Comprimés enrobés dosés à 25mg de principe actif dl 7-[(3-chloro 6-methyl 5,5-dioxo 6,11-dihydro dibenzo (c,f) thiazepin 1,2-11-yl) amino] heptanoate de sodium 26g2

| | |
|---|---|
| Amidon de maïs | 2g5 |
| Mannitol | 88g5 |
| Stéarate de magnésium | 2g5 |
| Talc | 6g5 |
| pour 1.000 comprimés | |

| Bicarbonate de sodium | |
|---|---|
| Carboxymethyl cellulose sodique | |
| Cire blanche | |
| Oléate de glycéryle. | |
| Oxyde de titane | qs pour enrober le |
| Polysorbate de polyéthylene glycol | noyau jusqu'à un poids |
| Polyvinyl pyrrolidone | unitaire de 0g170 |
| Saccharose | |
| Silice colloïdale | |
| Talc | |

EXEMPLE II

Comprimés enrobés à 12mg5 de principe actif par unité de prise.

dl 7- [(3-chloro 6-methyl 5,5-dioxo 6,11-dihydro dibenzo (c,f) thiazepin 1,2-11-yl) amino] heptanoate de sodium 13g1

| | |
|---|---|
| Amidon de maïs | 2g5 |
| Mannitol | 101g |
| Stéarate de magrésium | 2g5 |
| Talc | 6g5 |
| pour 1.000 comprimés | |

| Bicarbonate de sodium | |
|---|---|
| Carboxymethyl cellulose sodique | |
| Cire blanche | |
| Oléate de glycéryle | |
| Oxyde de titane | qs pour enrober le noyau |
| Polysorbate de polyethylene glycol | jusqu'à un poids de |
| Polyvinylpyrrolidone | 0g170 par comprimé |
| Saccharose | |
| Silice colloïdale | |
| Talc micronisé | |

0004516

- 9 -

<u>EXEMPLE III</u>

Suspension buvable dosée à 0g25 de principe actif pour 100ml

acide dl 7- [(3-chloro 6-methyl 5,5-dioxo 6,11-dihydro dibenzo (c,f) thiazepin 1,2-11-yl) amino] heptanoïque 0g250

| | |
|---|---|
| Saccharose | 5g |
| Sorbitol | 2g5 |
| p-hydroxybenzoate de methyle | 0g095 |
| p-hydroxybenzoate de propyle | 0g025 |
| Aromes | 0g005 |
| Gomme adragante | 0g45 |
| Eau purifiée   qsp 100ml | |

<u>EXEMPLE IV</u>

Effet normalisateur des compositions à base d'acide 7-[(3-chloro 6-methyl 5,5-dioxo 6,11-dihydrodibenzo (c,f) thiazepin 1,2-11-yl) amino] heptanoïque sur les troubles moteurs gastro-duodénaux en clinique humaine.

I - <u>BUT DE L'ETUDE</u> :

Evaluer l'efficacité du principe actif dans trois posologies différentes :

- 50 mg/jour de principe actif en 2 prises
- 25 mg/jour de principe actif en 1 ou 2 prises
- 12,5 mg/jour de principe actif en 1 prise et comparé au Placebo, à travers une étude clinique et électromyographique digestive externe, en double insu, chez 24 sujets atteints de <u>dyspepsie hyposthénique</u> en rapport avec un <u>état dépressif psychasthénique</u>.

II - MODALITES DE TRAITEMENT :

Les malades ont été divisés en deux groupes de 12 sujets

. Un ler groupe de 12 cas a reçu successivement et en cross-over 1 comprimé par jour de 12,5mg et de 25mg de PA. (Un malade de ce groupe a abandonné le traitement)

. Un 2ème groupe de 12 cas a reçu successivement et en cross-over 2 comprimés par jour, soit 2 x 12,5mg et 2 x 25mg de principe actif.

Durée du traitement :

Les passages de principe actif étaient chacun d'une durée de 15 jours et séparés par des passages de placebo d'une durée de 15 jours également.

Ordre de passage :

| | 2 semaines | 2 semaines | 2 semaines | 2 semaines |
|---|---|---|---|---|
| Sous-Groupe I ▷ 6 sujets | 1 x 25 mg (comprimés PA) | 1 x placebo | 1 x 12,5 mg (comprimés PA) | 1 x placebo |
| Sous-Groupe II ▷ 6 sujets | 1 x 12,5 mg (comprimés PA) | 1 x placebo | 1 x 25 mg (comprimés PA) | 1 x placebo |
| Sous-Groupe III ▷ 6 sujets | 2 x 12,5 mg (comprimé PA) | 2 x placebo | 2 x 25 mg (comprimé PA) | 2 x placebo |
| Sous-Groupe IV ▷ 6 sujets | 2 x 25 mg (comprimé PA) | 2 x placebo | 2 x 12,5 mg (comprimé PA) | 2 x placebo |

GR. I { Sous-Groupe I, Sous-Groupe II }
GR. II { Sous-Groupe III, Sous-Groupe IV }

EMG    T 0    T 1    T 2    T 3

Examen Clinique    t 0    t 1    t 2    t 3    t 4

PA = Comprimés renfermant l'acide 7-[(3-chloro 6-methyl 5,5-dioxo 6,11-dihydro dibenzo (c,f) thiazepin 1,2-11-yl) amino] heptanoïque.

L'appartenance des malades aux groupes se faisait par tirage au sort.

Les sujets ont donc pu recevoir les posologies suivantes :

. soit 50 mg/jour

. soit 25 mg/jour

. soit 12,5 mg/jour de principe actif

Tous les malades étaient ambulatoires.

III - CRITERES_D'APPRECIATION_DES_RESULTATS

Chaque sujet avait au minimum :

1. Un_syndrome_dyspeptique_hyposténique, avec

- ballonnements
- pesanteur
- somnolence post-prandiale
- brûlures et / ou douleurs épigastriques
- états nauséeux

2. Un_état_dépressif_psychasténique, avec

- dépression
- inhibition psycho-motrice
- asthénie
- anxiété psychique et / ou somatisée
- troubles du sommeil (difficulté à l'endormissement ou réveil précoce)

3. Un_électromyogramme_digestif_externe_pathologique, avec un retard d'apparition des ondes gastriques après le repas (d'au moins 2 heures).

IV - QUOTATION_DES_SYMPTOMES

. Selon la gravité clinique, de 0 à 3

. Pour l'électromyogramme digestif, chez un sujet normal, les ondes gastriques apparaissent dès l'ingestion d'un repas équilibré et persistent pendant 5 heures en moyenne. Cette situation était côtée 0.

Chez le dyspeptique hyposténique, il existe une absence d'ondes gastriques dans la phase post-prandiale, qui peut durer plus de 3 heures. Cette situation était côtée 2.
Si, après le traitement, la sidération diminuait, elle était côtée 1.

V - CRITERES_D'EXCLUSION

Ont été exclus de l'étude :

- les ulcères gastro-duodénaux
- les lésions sténosantes du tube digestif
- les hernies hiatales
- les gastrectomies
- les lithiases biliaires
- les colites
- les néoplasmes

L'examen clinique était répété après chaque passage du produit actif, ou de placebo, soit 5 fois par malade.

En ce qui concerne les tracés EMG, nous avions prévu quatre enregistrements par sujet : avant traitement, après chaque passage de Principe Actif, et après le dernier passage Placebo.

Pendant toute la durée de cette expérimentation, les malades ne recevaient aucune médication réputée active sur la motilité gastro-intestinale, en dehors des compositions pharmaceutiques renfermant de l'acide 7-[(3-chloro 6-methyl 5,5-dioxo 6,11-dihydro dibenzo (c,f) thiazepin 1,2-11-yl) amino] heptanoïque. Deux exemples de tracés EMG digestifs externes chez l'adulte sain, sans troubles digestifs montrent les catactéristiques suivantes :

a) Dans_la_période_pré-prandiale

Dans la période pré-prandiale, de 9 h à 12 h, quand le
sujet est à jeun, depuis la veille au soir, les tracés
électriques sont très pauvres et pratiquement plats.
Seules quelques ondes coliques (Co) rompent cette monotonie.

b) Dans_la_phase_post-prandiale

dès l'ingestion des aliments, on voit apparaître des
ondes gastriques (visibles sur la piste locale épigastrique 1-2 et sur la piste sagittale passant par l'hypocondre droit 8-13). (voir figure b).

- pistes locales abdominales :
  • épigastrique (1-2)
  • hypocondre droit (7-8)
  hypocondre gauche (9-10)
- pistes sagittales
  passant par :
  • hypocondre droit (8-13)
  • hypocondre gauche (9-14)
- piste
  électrogastroentérographique
  • bras droit - jambe gauche
  (21-24)

Sujet à jeun depuis la veille au soir

Figure a

période pré-prandiale

5 minutes après le repas

Figure b .

période post-prandiale

Ces ondes gastriques persistent 5 heures en moyenne après le repas sans discontinuité.

## VI - RÉSULTATS

### I - Activité clinique Globale

**A** | Groupe I |

Nombre_de_cas : 11

Posologie : 1 comprimé par jour de 12,5mg ou de 25mg
ou de placebo.

Durée_du_traitement : 15 jours pour chaque passage

1) Posologie à 1 x 12,5 mg/jour de principe actif
les compositions pharmaceutiques à 12,5 mg de principe actif ont apporté une très nette amélioration de l'ensemble des symptômes ; digestifs, psychiques, ainsi que des tracés EMG externes dans 5 cas sur 11, soit 45,4% de bonne activité et 54,6% d'activité moyenne dans 6 cas sur 11.

2) Posologie à 1 x 25 mg/jour de principe actif
A cette posologie, les compositions pharmaceutiques ont eu un très bon effet thérapeutique global dans 9 cas sur 11, soit dans 81,8 % des cas

L'absence d'effet a été notée dans 2 cas
soit chez 18,2 % des sujets.

3) Placebo 1er et 2e passages
Pendant les deux passages, et chez les mêmes malades, l'effet placebo s'est manifesté 2 fois sur 11, soit dans 18,1 % des cas
Dans les 9 autres cas, le placebo a été totalement inactif et ressenti comme un retour à la symptomatologie initiale.

**B** | Groupe II

Nombre_de_cas : 12

Posologie : 2 comprimés par jour de 12,5mg ou de 25mg ou le placebo.

Durée_du_traitement : 15 jours pour chaque passage.

1) Posologie à 2 x 12,5mg/jour
Bon effet thérapeutique global obtenu chez 8 malades sur 12, soit dans 66,6% des cas. L'effet a été moyen dans 4 cas, soit chez 33,3% des malades.

2) Posologie à 2 x 25mg/jour
A cette posologie, supérieure à celle préconisée en thérapeutique courante, l'activité a été bonne dans 10 cas sur 12, soit chez 83,3% des malades.

L'activité a été moyenne deux fois, soit chez 16,6% des malades. L'un de ces patients a abandonné l'essai parce que le produit était inefficace lors du 3e passage, soit à la posologie de 2 x 25mg/jour.

3) Placebo 1er passage

L'effet placebo a été présent 1 fois sur 12, soit chez 8,3% des malades de ce groupe.

Placebo 2e passage

Au 2e passage placebo, s'ajoutant au cas antérieur, un autre malade a abandonné l'essai en raison de l'inefficacité du produit.

Sur les 10 cas restants, l'effet placebo s'est manifesté 2 fois, soit dans 20% des cas.

Au total, sur l'activité globale, on voit que :

. Aux trois posologies utilisées (12,5mg, 25mg et 50mg), et dans les deux groupes de malades étudiés, le principe actif a toujours été nettement supérieur au placebo, démontrant ainsi son efficacité.

. Il existe toujours une relation directe entre la posologie employée et l'activité : plus la posologie est élevée, plus l'effet thérapeutique est important et concerne un plus grand nombre de cas

. Le rapport dose-activité devient plus évocateur encore, lorsque l'on regarde les résultats globaux selon la posologie et indépendamment du nombre de prises administrées (les 2 groupes de malades étant statistiquement comparables)

EXEMPLE V

Effet de la suspension buvable à base d'acide dl-7 [(3-chloro 6-méthyl 5,5-dioxo 6,11-dihydro dibenzo (c,f) thiazépine 1,2-11 yl) amino] heptanoïque sur les gastropathies.

I - BUT DE L'ETUDE

Evaluer l'efficacité du médicament selon l'invention comparativement à un placebo, pour traiter 38 malades atteints de gastrophatie psycho-fonctionelle.

- METHODE

a) Modalités_de_traitement

Les 38 malades ont été divisés en deux groupes séparés, déterminés par tirage au sort. Chaque groupe recevait un seul produit pendant le traitement.

. Le groupe I recevait le médicament à la posologie journalière de 25mg de principe actif administré en deux prises égales, soit une cuillerée contenant 12,5mg PA le matin et une à midi.

. Le groupe II recevait un placebo (une cuillerée le matin et une à midi).

Le traitement a été administré en monothérapie et pendant deux semaines.

b) Appréciation_des_résultats

L'efficacité du traitement a été jugée selon la gravité des principaux symptômes cliniques.

Chaque malade a fait l'objet d'une fiche d'observation complétée avant et en fin de traitement.

Ont été particulièrement notées deux catégories de symptômes :

. <u>Les symptômes digestifs</u> : douleurs abdominales, brûlures, ballonnements, nausées, éructations, sensation de diges- tion difficile, constipation.

. <u>Les symptômes psychiques</u> : dépression, anxiété, fatiga- bilité, baisse de l'activité, troubles du sommeil, somatisations cardio-vasculaires.

La cotation des symptômes a été établie, selon la gravité, de 0 à 3,

. 1 = symptôme d'intensité faible

. 2 à 3 = symptôme d'intensité forte.

Une appréciation de <u>l'acceptabilité</u> digestive, cardio- vasculaire et neurologique du médicament selon l'invention a été ensuite portée.

<u>La tolérance biologique</u> a été jugée à travers,

. un bilan sanguin (hémogramme complet)

. un bilan d'hémostase (TS. TC. TP.)

. un bilan hépatique avec dosage de ,
  - transaminases SGOT, SGPT
  - phosphatases alcalines

. un bilan rénal avec dosage de
  - créatinine plasmatique effectués avant et après deux semaines de traitement

Tous les sujets traités présentaient les signes classiques d'un syndrome dyspeptique et sans lésion organique décelable par fibroscopie ou examen radiologique effectués avant traitement.

On a classé les résultats globaux, selon l'efficacité, en trois groupes :

1. Les malades ayant obtenu un résultat bon ou excellent et dont la symptomatologie avait été, dans son ensemble, très améliorée ou abolie par la prise du médicament.

2. Un groupe classé comme <u>résultat moyen</u>, où le traite- ment a entraîné une diminution de la symptomatologie, tant digestive que psychique.

3. Un troisième groupe classé comme <u>résultat insuffisant</u>, pour lequel lemédicament ou le placebo n'ont manifesté aucun effet.

III - <u>RESULTATS</u>

A. <u>Activité clinique globale</u>

Groupe I traité par le médicament selon l'invention

<u>Nombre de cas</u> : 18 malades traités

Nous avons constaté,

. Une bonne activité (dont 2 excellentes) dans 10 cas soit chez 55,5% des malades.

. Une activité moyenne dans 1 cas, soit chez 5,5% des malades.

. Une activité insuffisante dans 7 cas, soit chez 39% des malades.

Groupe II - placebo

<u>Nombre de cas</u> : 20

Par rapport au groupe I recevant le médicament selon l'invention, on a obtenu,

. Une bonne activité dans 6 cas seulement (sans résultats excellents) soit chez 30% des malades.

. Une activité moyenne dans 2 cas, soit chez 10% des malades.

. Une activité insuffisante dans 12 cas, soit chez 60% des malades.

On voit donc que le médicament selon l'invention est nettement supérieur au placebo puisque l'on obtient 55,5% de bons résultats avec ce médicament et 30% avec le placebo où l'on note l'absence d'excellents résultats.

B. Activité selon les symptômes

Si on passe en revue les résultats thérapeutiques obtenus avec le médicament selon l'invention par rapport au placebo, dans chacun des symptômes suivants on constate :

a) SYMPTOMES DIGESTIFS

Les symptômes digestifs cibles ont été, les éructations, les ballonnements, les brûlures épigastriques, les nausées, la constipation ou la diarrhée et surtout la douleur épigastrique. Ce dernier symptôme occupe un large éventail des possibilités, depuis la douleur vague ou diffuse, jusqu'aux crampes intenses. Enfin, du fait de son extrême fréquence, la sensation de flatulence, de digestion difficile a été aussi étudiée.

b) DOULEURS EPIGASTRIQUES

Groupe I malades traités avec le médicament selon l'invention

Nombre_de_cas : 18 malades

Symptôme présent chez tous les malades, coté de forte intensité (de 2 à 3) chez 15 sujets et de faible intensité chez 3 autres.

. Le médicament selon l'invention a fait disparaître totalement les douleurs dans 10 cas, soit chez 55,5% des malades, parmi lesquels 9 présentaient des douleurs de forte intensité.

. Dans 2 cas, les douleurs ont été atténuées, soit chez 11% des malades.

Au total, l'activité du médicament selon l'invention sur des douleurs épigastriques fortes a été bénéfique chez 66,6% des malades.

Groupe II - placebo

Nombre de cas : 20

Symptôme retrouvé chez tous les malades, de forte intensité.

. Sous placebo, les douleurs disparaissent 5 fois, soit
chez 25% des malades

. Elles sont atténuées dans 3 autres cas, soit chez
15% des malades.

Ce qui représente un effet placebo dans 40% des cas.

L'intensité de l'effet du médicament selon l'invention
est double de celle obtenue avec le placebo($\Delta = 62\%$
et 34% pour le placebo).

c) ERUCTATIONS

Groupe I, malades traités avec le médicament selon
l'invention

Nombre de cas : 7 malades

Symptôme présent dans 7 cas, de moyenne intensité.

Sous l'effet du médicament selon l'invention, les
éructations disparaissent dans 5 cas, soit chez 71,4%
des malades.

Ce résultat est significatif sur le plan stastitique
(test t = 3,286 ; p < 0,02).

En conclusion, le médicament selon l'invention s'est
montré très actif sur les éructations et très supérieur au placebo, faisant disparaître le symptôme
dans trois quarts des cas, et avec une forte intensité thérapeutique ($\Delta = 67\%$). Le placebo n'agit que
dans 43% des cas, et avec une intensité beaucoup plus
faible ($\Delta = 30\%$).

La supériorité du médicament selon l'invention existe
également sur le plan de la significativité stastique.

d) PESANTEUR (sensation de digestion difficile)

Groupe I, malades traités avec le médicament selon
l'invention Symptôme présent chez 11 malades, et de moyenne intensité.

Sous l'effet du médicament selon l'invention, la pesanteur disparaît chez 4 malades, soit dans 36,3% des cas et s'atténue chez 1 malade, soit dans 9% des cas.

Ce qui représente 45% d'activité bénéfique.

Le résultat est significatif sur le plan statistique (test t = 2,886 ; p < 0,02 ; $\Delta$ = 38%)

Le placebo n'a eu qu'un effet limité, puisque, à la fin du traitement, nous constatons,

. 2 disparitions du symptôme, soit chez 15,3% des malades

. 2 améliorations, soit chez 15,3% des malades

L'effet placebo, qui s'est exercé chez 30,6% des malades, n'est pas significatif sur le plan statistique (test t = 2,132 ; p < 0,10 ; $\Lambda$ = 29%).

Le médicament selon l'invention se montre donc supérieur au placebo quant à son activité sur ce symptôme et donne des résultats statistiquement significatifs. L'effet exercé est plus intense pour le médicament que pour le placebo.

b) BRULURES EPIGASTRIQUES

Groupe I, malades traités avec le médicament selon l'invenrion.

Nombre de cas : 13

On relève des brûlures de forte intensité dans 10 cas et de faible intensité dans 3 autres cas présentes avant traitement.

Sous traitement par le médicament selon l'invention,

. le symptôme disparaît totalement chez 9 malades, dont 8 avaient ressenti des brûlures de forte intensité, soit chez 69% des patients.

Cette bonne activité sur les brûlures épigastriques, retrouvée dans 69% des cas, est, sur le plan statistique, très significative (test t = 3,988 ; p < 0,001 ; l'intensité de l'effet s'élève à un Δ = 71%).

L'effet placebo, assez élevé, est significatif sur le plan statistique (test t = 3,222 : p < 0,01).

Toutefois, il faut noter que les compositions selon l'invention donnent lieu à de bons résultats dans 69% des cas traités, alors que le placebo ne fournit de résultats que dans 31% des cas.

L'intensité de l'effet exercé par le médicament selon l'invention (le Δ) s'élève à 71% et celle du placebo à 38% seulement.

f) BALLONNEMENTS

Groupe I, malades traités par le médicament selon l'invention :

Nombre de cas : 13 malades

Les ballonnements étaient, avant traitement, de forte intensité chez 6 malades et de faible intensité chez 7 autres malades.

Le médicament selon l'invention provoque :

. la disparition des ballonnements dans 2 cas, soit chez 15,4% des malades

. Leur amélioration dans 4 cas, soit chez 30,7% des malades.

L'activité du médicament selon l'invention s'exerce donc sur 46% des malades ayant présenté ce symptôme. Notons que 4 malades sur les 6 souffrant de ballonnements importants post-prandiaux ont été soulagés par le produit.

Le résultat est très significatif sur le plan statistique (test t = 3,207 ; p < 0,01 ; Δ = 31%).

Groupe II, placebo Nombre_de_cas : 14 malades

Parmi les 14 malades de ce groupe, 6 souffraient de ballonnements post-prandiaux importants et 8 de faible intensité.

L'effet placebo s'est exercé sur 6 malades avec,

. disparition du symptôme dans 2 cas, soit chez 14,2% des malades.

. amélioration dans 4 cas, soit chez 28,5% des malades;

g) NAUSEES

Groupe I, malades traités par le médicament selon l'invention.

Dans ce groupe, 5 malades présentaient des nausées importantes (cotées 2 à 3) et les 9 autres malades des nausées de faible intensité.

Ainsi, on note après traitement,

. la totale disparition des nausées dans 9 cas, dont trois de forte intensité, soit un très bon effet chez 64,3% des malades.

. Une amélioration dans 1 cas, soit chez 7% des malades

Ce qui représente un pourcentage de 71,3% de bons et très bons résultats.

Aucune aggravation n'a été constatée sous traitement.

La bonne activité sur les nausées touchait 4 malades graves sur 5. L'effet exercé est très intense ($\Delta = 64\%$).

Ce résultat est hautement significatif sur le plan statistique (test t = 4,759 ; p < 0,001).

Groupe II, placebo

Nombre_de_cas : 17

4 cas présentaient avant traitement des nausées importantes (cotées 2), les 13 autres malades ressentant des nausées de faible intensité. L'effet placebo s'est exercé chez 29,3% des malades.

Le médicament selon l'invention s'est avéré être un très bon agent anti-nauséeux, nettement supérieur au placebo sur les plans des pourcentages d'activité, de l'intensité de l'effet et de la significativité statistique des résultats. Il améliore les nausées les plus graves.

h) CONSTIPATION

Groupe I, malades traités par le médicament selon l'invention.

Nombre de cas : 11 malades

On note une constipation importante chez 4 malades, avant traitement et de faible intensité chez 7 autres malades.

. Le médicament selon l'invention a amélioré le symptôme qui était de forte intensité avant traitement chez 3 sujets, soit dans 27% des cas.

. Mais, ce qui est le plus important, il n'a jamais aggravé ou provoqué une constipation, ce qui est la preuve d'une bonne tolérance.

Le médicament selon l'invention soulage la constipation, ne l'aggrave pas, et surtout ne la provoque pas (expression de sa bonne tolérance).

CONCLUSIONS

Sur l'ensemble de sept symptômes digestifs cibles des 38 malades dyspeptiques étudiés, on constate que le médiment selon l'invention a une activité nettement supérieure à celle du placebo dans six symptômes sur sept.

1. L'effet maximum obtenu dans le groupe I recevant le médicament selon l'invention a dépassé de plus de 50% celui obtenu dans le groupe soumis à un placebo, 5 fois sur 6 (exception : la constipation où le médicament selon l'invention, de même que le placebo, n'ont eu que des effets partiels).

2. Il faut mettre en relief le très bon effet des compositions selon l'invention sur les nausées, les éructations, les brûlures et les douleurs épigastriques.

3. Les compositions selon l'invention agissent avec une grande intensité thérapeutique, et sur les cas les plus graves.

Ainsi, le pourcentage d'amélioration apportée par les compositions selon l'invention par rapport à la gravité des symptômes constatée avant le traitement, est très supérieur au placebo - 5 fois sur 7 - (douleurs, éructations, brûlures, nausées, constipation) et une fois légèrement supérieur (pesanteur).

Dans un seul symptôme, les ballonnements, le pourcentage n'est pas supérieur au placebo.

| SYMPTOMES | Pourcentage d'amélioration ( $\Delta$ ) | |
|---|---|---|
| | MEDICAMENT | PLACEBO |
| DOULEURS EPIGASTRIQUES | 62 % | 34 % |
| ERUCTATIONS | 67 % | 40 % |
| PESANTEUR | 38 % | 29 % |
| BRULURES EPIGASTRIQUES | 71 % | 38 % |
| BALLONNEMENTS | 31 % | 28 % |
| NAUSEES | 64 % | 23 % |
| CONSTIPATION | 19 % | 9 % |

IV - ACCEPTABILITE

L'acceptabilité des compositions selon l'invention a été appréciée selon des critères cliniques et biologiques.

a) Acceptabilité clinique

Les effets des compositions selon l'invention sur l'appareil digestif, sur le système nerveux, ainsi que d'éventuels troubles allergiques ont été systématiquement recherchés.

b) <u>Acceptabilité biologique</u>

Un bilan biologique avant et après traitement, a été réalisé chez tous les malades. Il comprenait :

- un hémogramme complet, avec numération des GR., GB., des plaquettes et formule sanguine.

- un bilan d'hémostase (TP., TS., et TC.)

- dosages des transaminases SGOT., SGPT., phosphatases alcalines et de la créatinine plasmatique.

<u>RESULTATS</u>

a) <u>Acceptabilité clinique</u> des compositions selon l'invention

·Globalement, ces compositions ont entraîné une excellente acceptabilité.

Elle a été jugée,

. excellente chez 17 malades (soit 94,4%)

. moindre chez 1 malade (soit 5,6%), qui a présenté une céphalée cédant à l'arrêt du traitement.

SCIENCE UNION ET Cie, SOCIETE FRANCAISE DE RECHERCHE MEDICALE
14, rue du Val d'Or  92150 SURESNES (France)

----------------

Nouvelles compositions pharmaceutiques agissant sur la
motricité gastro-intestinale

----------------

R e v e n d i c a t i o n s

1) Nouvelles compositions pharmaceutiques agissant sur la motricité gastro-intestinale caractérisées en ce qu'elles renferment comme principe actif l'acide 7-[(3-chloro 6-methyl 5,5-dioxo 6,11-dihydro dibenzo (c,f) thiazepin 1,2-11-yl) amino] heptanoïque sous forme racémique ou sous forme dédoublée, ou un de ses sels en association avec un véhicule ou un excipient inerte, non-toxique, pharmaceutiquement acceptable.

2) Jne composition pharmaceutique selon la revendication 1 dans laquelle le principe actif est l'acide dl 7-[(3-chloro 6-methyl 5,5-dioxo 6,11-dihydro dibenzo (c,f) thiazepin 1,2-11-yl) amino] heptanoïque.

3) Une composition pharmaceutique selon la revendication 1 dans laquelle le principe actif est un sel de base minérale ou organique de l'acide dl 7-[(3-chloro 6-methyl 5,5-dioxo 6,11-dihydro dibenzo (c,f) thiazepin 1,2-11-yl) amino] heptanoïque.

4) Une composition pharmaceutique selon la revendication 1 dans laquelle le sel de base minérale ou organique de l'acide dl 7-[(3-chloro 6-methyl 5,5-dioxo 6,11-dihydro dibenzo (c,f) thiazepin 1,2-yl) amino] heptanoïque est le sel de sodium

5) Une composition pharmaceutique selon la revendication 1 dans laquelle le principe actif est un sel de l'acide dl 7-[(3-chloro 6-methyl 5,5-dioxo 6,11-dihydro dibenzo (c,f) thiazepin 1,2-11-yl) amino] heptanoïque avec un acide minéral ou organique thérapeutiquement acceptable.

6) Une composition pharmaceutique selon l'une des revendications précédentes dans laquelle la teneur en principe actif s'échelonne entre 12mg et 50mg exprimé en acide 7-[(3-chloro 6-methyl 5,5-dioxo 6,11-dihydro dibenzo (c,f) thiazepin 1,2-11-yl) amino] heptanoïque.

7) Une composition selon l'une des revendications précédentes dans laquelle le véhicule ou l'excipient inerte, non toxique est un de ceux qui conviennent pour l'administration par voie parentérale, buccale, ou rectale.